# EUROPÄISCHE PATENTSCHRIFT

(11) **EP 0 513 055 B1**
(45) Veröffentlichungstag und Bekanntmachung des Hinweises auf die Patenterteilung: **07.09.1994**
(21) Anmeldenummer: 91902698.9
(22) Anmeldetag: 24.01.1991
(51) Int. Cl.: C07C 67/14, C07C 51/04, C07C 69/63, C07C 53/16

(54) **VERFAHREN ZUR UMSETZUNG EINER NIEDERMOLEKULAREN HYDROXIVERBINDUNG MIT EINEM CARBONSÄUREHALOGENID**
PROCESS FOR REACTING A LOW MOLECULAR WEIGHT HYDROXY COMPOUND WITH A CARBOXYLIC ACID HALIDE
PROCEDE POUR EFFECTUER LA REACTION D'UN COMPOSE HYDROXY DE FAIBLE POIDS MOLECULAIRE AVEC UN HALOGENURE D'ACIDE CARBOXYLIQUE

(30) Priorität: 02.02.1990 DE 4003014
(43) Veröffentlichungstag der Anmeldung: 19.11.1992
(73) Patentinhaber: HOECHST AKTIENGESELLSCHAFT, 65926 Frankfurt am Main (DE)
(72) Erfinder: MILTENBERGER, Karlheinz, D-8906 Gersthofen (DE)
(86) Internationale Anmeldenummer: EP9100131
(87) Internationale Veröffentlichungsnummer: WO9111423

(56) Entgegenhaltungen:
- DE-C- 835 888
- HOUBEN-WEYL, "Methoden der organischen Chemie, Georg Thieme Verlag, DE, 4. Auflage, Band VIII, Sauerstoffverbindungen III, 1952, S. 543-547
- HOUBEN-WEYL, "Methoden der organischen Chemie", Georg Thieme Verlag, DE, Erweiterungs- und Folgebände zur 4. Auflage, Band E5, Carbonsäuren und Carbonsäurederivate, 1985, S. 219-221

## Beschreibung

Die Erfindung bezieht sich auf ein Verfahren zur umweltfreundlichen Umsetzung einer niedermolekularen Hydroxiverbindung mit einem aliphatischen Carbonsäurehalogenid, insbesondere von Alkoholen mit Säurechloriden.

Bei der Herstellung von Estern aus Säurechloriden und Hydroxiverbindungen wird üblicherweise die alkoholische Komponente im Reaktionsgefäß vorgelegt und das Säurechlorid nach Maßgabe der zulässigen Temperatur- und Druckverhältnisse zudosiert. Bei dieser Arbeitsweise entwickelt sich HCl-Gas, das sich zunächst in der alkoholischen Komponente unter Wärmeentwicklung löst. Erst nach eingetretener Sättigung wird HCl-Gas freigesetzt. Der Vorteil dieser Verfahrensweise besteht darin, daß eine unkontrollierbare Reaktion des Säurechlorids mit unerwünschten Hydroxiverbindungen, wie beispielsweise Wasser bei einem Kühlerbruch, ausgeschlossen werden kann. Der wesentliche Nachteil dieser Arbeitsweise ist, daß das bei der Umsetzung entstehende HCl-Gas im vorgelegten Alkohol unter Freisetzung einer erheblichen Lösungswärme absorbiert wird und mit der alkoholischen Komponente unerwünschte Nebenreaktionen eingeht. Im Falle der Herstellung von Dichloressigsäuremethylester aus vorgelegtem Methanol und dosiert zugegebenen Dichloracetylchlorid entsteht in einer temperaturabhängigen Nebenreaktion Methylchlorid und Wasser. Bereits bei einer Temperatur wenig über 40°C bilden sich erhebliche Mengen des wegen seiner umweltbelastenden Eigenschaften unerwünschten Methylchlorids. Aber auch bereits im Temperaturbereich unter 40°C kann im entweichenden HCl-Gas Methylchlorid nachgewiesen werden, welches eine spezielle Abgas-Reinigung erforderlich macht.

Das bei dieser Nebenreaktion ebenfalls entstehende Wasser führt bei der Aufarbeitung eines solchen Reaktionsgemisches, insbesondere in Gegenwart leicht verseifbarer Ester, wie z.B. Dichloressigsäuremethylester, zu einer unerwünschten Verseifung, d.h. zu einer unter Umständen erheblichen Minderung der Ester-Ausbeute.

Es wurde nunmehr gefunden, daß die geschilderten Nachteile bei der Herstellung von Estern aus Säurehalogeniden und niedermolekularen Hydroxiverbindungen vermieden werden können, wenn man in der Weise verfährt, daß man das Säurehalogenid vorlegt und die Hydroxiverbindung zudosiert.

Die Erfindung betrifft somit das in den Ansprüchen beschriebene Verfahren.

Nach dem erfindungsgemäßen Verfahren werden niedermolekulare Hydroxiverbindungen mit Säurehalogeniden umgesetzt. Geeignete Hydroxiverbindungen sind Wasser, aliphatische C₁-C₁₂-Alkohole, cycloaliphatische C₆-C₁₂-Alkohole und aromatische Alkohole. Vorzugsweise werden Wasser, aliphatische C₁-C₁₂-Alkohole und cycloaliphatische C₆-C₁₂-Alkohole eingesetzt, insbesondere Wasser und aliphatische C₁-C₄-Alkohole, beispielsweise Methanol, Ethanol, n- und i-Propanol, Butanole.

Geeignete Carbonsäurehalogenide sind die Fluoride, Chloride und Bromide, vorzugsweise Bromide und Chloride von aliphatischen C₂-C₆-Carbonsäuren, cycloaliphatischen C₆-C₁₂-Carbonsäuren und aromatischen Carbonsäuren. Vorzugsweise werden die Chloride der niederen aliphatischen C₂-C₆-Halogencarbonsäuren, wie beispielsweise Chloressigsäuren oder Chlorpropionsäuren eingesetzt.

Die Umsetzung der Reaktionspartner erfolgt in der Weise, daß das Carbonsäurehalogenid während der Reaktion im Überschuß gehalten wird und die Hydroxiverbindung zum Säurehalogenid gegeben wird. Zu diesem Zweck werden 5 bis 20 mol-% der gesamten für die Umsetzung vorgesehenen Menge Säurehalogenid im Reaktionsgefäß vorgelegt und dann das restliche Säurehalogenid und die Hydroxiverbindung in nahezu stöchiometrischem Verhältnis nach und nach mit einer Geschwindigkeit zugeführt, die von der Abführung des Halogenwasserstoffs und der Reaktionswärme abhängig ist. Am Schluß, nach Verbrauch der nachzuführenden Menge Säurehalogenid, wird auch der im Reaktionsgefäß verbliebene Rest an Säurehalogenid umgesetzt. Die Hydroxiverbindung wird in einer Menge von 105 bis 120 mol-%, bezogen auf das gesamte Säurehalogenid, in das Reaktionsgefäß eingegeben.

Falls die Reaktionspartner flüssig sind, werden sie vorzugsweise in unverdünnter Form in Abwesenheit eines Lösemittels zusammengebracht. Feste Reaktionspartner werden zweckmäßigerweise vor der Reaktion in einem inerten Lösemittel gelöst, beispielsweise in einem aliphatischen, cycloaliphatischen oder aromatischen Kohlenwasserstoff, in Tetrachlormethan oder in anderen inerten halogenierten Kohlenwasserstoffen, beispielsweise in Trichlorethen.

Die Reaktionstemperatur ist vom Druck und von den Reaktanten abhängig und beträgt 20 bis 100, vorzugsweise 20 bis 50°C, im Falle der Umsetzung von Säurechloriden bis 40°C. Der Druck beträgt 1 bis 3, vorzugsweise 1 bis 1,5 bar.

Als Reaktionsgefäß kann jeder für Veresterungsreaktionen geeignete Apparat verwendet werden, beispielsweise Kolben und Kessel mit Rührern, Rückflußkühler, Zulaufgefäßen und Kontroll- und Steuereinrichtungen.

Das entstandene Reaktionsprodukt, Säure oder Ester, wird nach allgemein bekannten Methoden wie Destillation, Kristallisation oder einem anderen Verfahren in reiner Form gewonnen. In manchen Fällen kann das Reaktionsprodukt ohne weitere Reinigungsoperation verwendet werden.

Durch das erfindungsgemäße Verfahren tritt praktisch keine Lösungswärme auf, welche abgeführt werden muß, da der durch die Reaktion entstehende Halogenwasserstoff in dem Säurehalogenid wenig löslich ist. Der Halogenwasserstoff entweicht vom Beginn der Reaktion an. Nach diesem Verfahren lassen sich auch Säuren, die auf anderem Wege nur schwer erhältlich sind, in guter Ausbeute aus ihren Säurechloriden gewinnen.

Bei Glasapparaturen führt ein eventueller Bruch des Rückflußkühlers und Eindringen von Kühlwasser in den Reaktionsraum nicht zu einem Durchgehen der Reaktion.

Die nachfolgenden Beispiele sollen die Erfindung erläutern.

### Beispiel 1

### Herstellung von Dichloressigsäureethylester (DEE)

In einem 2 dm³ -4-Halskolben mit Innenthermometer, Intensivkühler, Rührer, Claisenaufsatz und 2 Tropftrichtern á 500 cm³ wurden 500 cm³ Dichloracetylchlorid (DAC) (entsprechend 766,5 g = 5,20 mol) mit 319 cm³ absolutiertem Ethanol (entsprechend 251,7 g = 5,46 mol) umgesetzt. Für die Umsetzung wurden im Kolben bei laufendem Rührer 10 cm³ DAC vorgelegt und aus den beiden Tropftrichtern DAC und Ethanol kontinuierlich zutropfen gelassen, wobei der Überschuß an DAC bis zum Reaktionsende beibehalten wurde. Die Zutropfgeschwindigkeit war so einreguliert, daß in etwa 1,5 Minuten 10 cm³ DAC mit jeweils 6,1 cm³ Ethanol in etwa stöchiometrischen Verhältnissen reagierten. Während der Reaktion wurde der Ansatz gekühlt, damit die Innentemperatur nicht über 40°C anstieg. Das bei der Reaktion sich entwickelnde HCl-Gas wurde über den Intensivkühler abgeführt.

Gegen Reaktionsende, als das gesamte DAC zugelaufen war, befanden sich im anderen Tropftrichter noch ca. 18 - 24 cm³ Ethanol, die zur Umsetzung des DAC-Überschusses mit einem molaren Überschuß vom 5 % in den Kolben gegeben wurden.

Während der Umsetzung selbst mußte die DAC-Ethanol-Zugabe laufend überwacht werden, wobei die zudosierte Ethanolmenge gegenüber dem vorgegebenen Wert nur um höchstens ± 3 cm³ schwanken durfte. In Praxis bedeutet das, daß in gleichen Zeiträumen die zugegebene Ethanolmenge etwa 55 - 60 % der an den Tropftrichtern ablesbaren DAC-Menge entsprach.

Nach beendeter Reaktion wurde zur vollständigen Umsetzung und zur Entfernung des HCl-Gases der Ansatz noch etwa eine 1/4 Stunde gerührt. Anschließend wurde der entstandene Dichloressigsäureeethylester zur Reinigung einer Vakuumdestillation unterworfen. Nach der Vakuumdestillation wurden 778 g DEE erhalten. Das sind 95,3 % Ausbeute, bezogen auf DAC.

### Beispiel 2

### Herstellung von Trichloressigsäureethylester (TEE)

Analog der Herstellung von Dichloressigsäureethylester wurde auch Trichloressigsäureethylester (TEE) in der gleichen Apparatur hergestellt.
Hierzu wurden 500 cm³ Trichloracetylchlorid (TAC) (entsprechend 812,5 g = 4,47 mol) mit 274 cm³ absolutiertem Ethanol (entsprechend 216,3 g = 4,69 mol) umgesetzt. Die Umsetzung erfolgte wiederum so, daß 10 cm³ TAC vorgelegt und bis zum Reaktionsende als Überschuß beibehalten wurden. Aus den beiden Tropftrichtern wurden TAC und Ethanol kontinuierlich zutropfen gelassen, wobei jeweils 10 cm³ TAC mit 5,2 cm³ Ethanol in etwa stöchiometrischen Verhältnissen reagierten. Die zudosierte Ethanolmenge durfte gegenüber dem vorgegebenen Wert nur um höchstens ± 2,5 cm³ schwanken.
Während der Reaktion wurde gekühlt und das HCl-Gas über den Intensivkühler abgeführt.
Als das gesamte TAC zugelaufen war, befanden sich im Ethanol-Tropftrichter noch ca. 16 - 21 cm³ Ethanol, die als Überschuß zur Umsetzung des TAC-Überschusses zugegeben wurden.
Nach beendeter Reaktion wurde der Ansatz noch etwa eine 1/4 Stunde gerührt. Anschließend wurde der Trichloressigsäureethylester zur Reinigung einer Vakuumdestillation unterworfen.

Dabei wurden 778 g TEE erhalten, das sind 91 % Ausbeute, bezogen auf TAC.

### Beispiel 3

### Herstellung von Trichloressigsäure-n-butylester (TBE)

Trichloressigsäurebutylester wurde analog Beispiel 1 dargestellt. In der beschriebenen Apparatur wurden 500 cm³ TAC (812,5 g entsprechend 4,47 mol) mit 429,4 cm³ n-Butanol (347,8 g entsprechend 4,69 mol) umgesetzt. Die Umsetzung erfolgte wiederum so, daß 10 cm³ TAC vorgelegt und jeweils in gleichen Zeiträumen 10 cm³ TAC und 8,2 cm³ n-Butanol zudosiert wurden. Die zudosierte Butanol-Menge durfte gegenüber dem vorgegebenen Wert nur um höchstens ± 4 cm³ schwanken.
Nach Reaktionsende wurde das restliche n-Butanol, etwa 25 - 33 cm³, zugegeben, der Ansatz eine Viertelstunde gerührt und der entstandene Ester im Vakuum destilliert. Die Ausbeute betrug 915 g TBE. Das sind 93,3 %, bezogen auf TAC.

### Beispiel 4

### Herstellung von Dichloressigsäuremethylester (DME)

In einem 4 m³-Rührkessel wurden 2590 dm³ DAC (entsprechend 4000 kg = 27,14 kmol) mit 1150 dm³ Methanol (entsprechend 910 kg = 28,4 kmol) umgesetzt.
Hierzu wurden bei laufendem Rührer 30 dm³ DAC im Rührkessel vorgelegt und anschließend über zwei Dosierpumpen, die über eine gemeinsame Antriebswelle von einem Motor angetrieben wurden, Methanol und DAC stöchiometrisch zudosiert.
Die Zudosierung erfolgte über Zähler und Rotameter. Sie wurde so betrieben, daß in dem gleichen Zeitraum, in welchem 40 dm³ DAC eingespritzt wurden, auch 17 dm³ Methanol zudosiert wurden. Diese Mengen wurden an den Dosierpumpen eingestellt und laufend überwacht. Die dosierte Methanol-Menge durfte dabei nur um höchstens ± 8 dm³ gegenüber dem vorgegebenen, aufsummierten Sollwert schwanken. Dabei reagierte das Methanol mit dem DAC unter leicht exothermer Wärmetönung zu DME. Über eine Mantelkühlung wurde die Reaktionswärme abgeführt. Die Temperatur stieg nicht über 40°C.

Da im Reaktor während der ganzen Reaktion ca. 30 dm³ DAC im Überschuß vorhanden waren, kam es von Anfang an zu einer lebhaften HCl-Gasentwicklung. Im vorliegenden Fall war der Rührkessel mit einer Kolonne und aufgesetzten Glaskühlern versehen, über die das HCl-Gas entwich. Eventuell mitgerissenes Produkt wurde in den Kühlern zurückgehalten und tropfte wieder in den Reaktionsbehälter zurück. Aus Sicherheitsgründen wurde die Anlage so betrieben, daß der HCl-Gasdruck in der Anlage nicht über 1,2 bar anstieg. Nach etwa 7,5 Stunden war die gesamte DAC-Menge von 2590 dm³ (einschließlich der vorgelegten 30 dm³) "DAC" mit 1100 dm³ Methanol umgesetzt. Dies machte sich dadurch bemerkbar, daß die HCl-Entwicklung aufhörte und der Druck in der Anlage abfiel. Zur vollständigen Umsetzung wurden noch 50 dm³ Methanol nachdosiert und der gesamte Ansatz 15 Minuten gerührt.
Das Reaktionsgemisch wurde anschließend unter Vakuum destilliert. Dabei wurden 3650 kg DME erhalten. Das sind 94,3 % Ausbeute, bezogen auf DAC.

### Beispiel 5

### Herstellung von Dichloressigsäure (DIS)

Analog der DME-Herstellung wurden in dem gleichen 4 m³-Rührkessel 2590 dm³ DAC (entsprechend 4000 kg = 27,14 kmol) mit 540 dm³ Wasser (540 kg = 30 kmol) umgesetzt. Hierzu wurden wieder 30 dm³ DAC im Rührkessel vorgelegt. Das restliche DAC wurde anschließend mit dem Wasser stöchiometrisch zudosiert.
Die Zudosierung erfolgte analog über Zähler und Rotameter. Sie wurde so betrieben, daß in dem gleichen Zeitraum, in welchen jeweils 40 dm³ DAC zudosiert wurden, auch 7,5 dm³ Wasser eingespritzt wurden. Die dosierte Wassermenge durfte dabei höchstens um ± 3 dm³ gegenüber dem vorgegebenen, aufsummierten Sollwert schwanken; sie wurde laufend überprüft. Der DAC-Überschuß von 30 dm³ wurde bis zum Reaktionsende beibehalten. Nach ca. 7 Stunden war die gesamte DAC-Menge von 2510 dm³ (einschließlich der vorgelegten 30 dm³) mit 490 dm³ Wasser umgesetzt. Zur vollständigen Umsetzung wurden noch 50 dm³ Wasser nachdosiert und der Ansatz 15 Minuten gerührt. Bei der anschließenden Vakuumdestillation wurden 3390 kg Dichloressigsäure erhalten. Das sind 96,8 % Ausbeute, bezogen auf DAC.

## Patentansprüche

1. Verfahren zur Umsetzung einer niedermolekularen Hydroxiverbindung mit einem Carbonsäurehalogenid, dadurch gekennzeichnet, daß 5 bis 20 mol-% der gesamten für die Umsetzung vorgesehenen Menge Carbonsäurehalogenid im Reaktionsgefäß vorgelegt, das restliche Säurehalogenid und Hydroxiverbindung in nahezu stöchiometrischem Verhältnis nach und nach zugeführt und am Schluß, nach Verbrauch der nachzuführenden Menge Carbonsäurehalogenid, der im Reaktionsgefäß verbliebene Rest an Carbonsäurehalogenid mit der Hydroxiverbindung umgesetzt wird und die Hydroxiverbindung in einer Menge von 105 bis 120 mol-% bezogen auf die Gesamtmenge an Carbonsäurehalogenid eingesetzt wird.

2. Verfahren nach Anspruch 1, dadurch gekennzeichnet, daß die niedermolekulare Hydroxiverbindung Wasser oder ein aliphatischer C₁-C₁₂-Alkohol ist.

3. Verfahren nach Anspruch 1, dadurch gekennzeichnet, daß das Carbonsäurehalogenid ein Chlorid einer aliphatischen C₂-C₆-Halogencarbonsäure ist.

## Claims

1. A process for the reaction of a low-molecular hydroxyl compound with a carboxylic acid halide, wherein 5 to 20 mol% of the total amount of carboxylic acid halide intended for the reaction is initially introduced into the reaction vessel, the remaining acid halide and the hydroxyl compound are gradually introduced in approximately stoichiometric proportions and, at the end, after the amount of carboxylic acid halide to be added has been consumed, the carboxylic acid halide remaining in the reaction vessel is reacted with the hydroxyl compound, the hydroxyl compound being used in an amount of 105 to 120 mol% based on the total amount of carboxylic acid halide.

2. A process as claimed in claim 1, wherein the low-molecular hydroxyl compound is water or an aliphatic C₁-C₁₂ alcohol.

3. A process as claimed in claim 1, wherein the carboxylic acid halide is a chloride of an aliphatic C₂-C₆ halogenocarboxylic acid.

## Revendications

1. Procédé pour la réaction d'un composé hydroxy de bas poids moléculaire avec un halogénure d'acide carboxylique, caractérisé en ce qu'on introduit tout d'abord dans le réacteur 5 à 20 moles-% de la quantité totale d'halogénure d'acide carboxylique prévue pour la réaction, le reste d'halogénure d'acide et le composé hydroxy dans un rapport presque stoechiométrique sont amenés progressivement et finalement, après que la quantité d'halogénure d'acide carboxylique à amener ultérieurement soit consommée, on fait réagir le reste d'halogénure d'acide carboxylique restant dans le réacteur avec le composé hydroxy et on utilise le composé hydroxy dans une quantité de 105 à 120 moles-% par rapport à la quantité totale d'halogénure d'acide carboxylique.

2. Procédé selon la revendication 1, caractérisé en ce que le composé hydroxy de bas poids moléculaire est l'eau ou un alcool en C₁-C₁₂ aliphatique.

3. Procédé selon la revendication 1, caractérisé en ce que l'halogénure d'acide carboxylique est un chlorure d'un acide halogéno-carboxylique en C₂-C₆ aliphatique.
